# FASCICULE DE BREVET EUROPEEN

(11) **EP 1 415 642 B1**
(45) Date de publication et mention de la délivrance du brevet: **13.12.2006**
(21) Numéro de dépôt: 03291622.3
(22) Date de dépôt: 01.07.2003
(51) Int. Cl.: A61K 8/34, A61K 8/49, A61K 8/66, A61Q 5/10

(54) **Composition de teinture des fibres kératiniques contenant un prècurseur d'aldéhyde, une enzyme et une hydrazone**
Mittel zum Färben von Keratinfasern enthaltend einen Aldehydvorprodukten, ein Enzym und eine Hydrazone
Composition for dyeing keratinous fibres containing an aldehyde precursor, an enzyme and a hydrazone

(30) Priorité: 05.07.2002 FR 0208493
(43) Date de publication de la demande: 06.05.2004
(73) Titulaire: L'ORÉAL, 75008 Paris (FR)
(72) Inventeur: Burgaud, Hervé, 77230 Damartin en Geole (FR); Pereira, Rui, 77140 Montevrain (FR); Belcour-Castro Béatrice, 94340 Joinville le Pont (FR)
(74) Mandataire: Casalonga, Axel

(56) Documents cités:
- WO-A-01/47478
- WO-A-01/68042
- WO-A-02/47633
- DE-A- 2 334 738
- DE-A- 19 949 033
- FR-A- 2 769 219
- FR-A- 2 822 062
- US-A- 3 634 013
- HUENIG S ET AL: "AZOFARBSTOFFE DURCH OXYDATIVE KUPPLUNG II SYNTHESE KUPPLUNGSFAEHIGER HETEROCYCLISCHER 2-HYDRAZONE" JUSTUS LIEBIGS ANNALEN DER CHEMIE, VERLAG CHEMIE, WEINHEIM,, DE, vol. 609, 1957, pages 160-172, XP001058975 ISSN: 0075-4617

## Description

La présente invention concerne le domaine de la teinture des fibres kératiniques, elle concerne plus particulièrement une composition contenant un précurseur d'aldéhyde, une enzyme et une hydrazone hétéroaromatique capable de générer une substance colorée par réaction avec un aldéhyde, ainsi qu'un procédé de teinture des fibres kératiniques mettant en oeuvre cette composition et un dispositif à plusieurs compartiments contenant cette composition.

Il est connu de teindre les fibres kératiniques et en particulier les cheveux humains avec des compositions tinctoriales contenant des précurseurs de colorant d'oxydation, appelés généralement bases d'oxydation, tels que des ortho ou paraphénylènediamines, des ortho ou paraaminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des composés indoliques.

La variété des molécules mises en jeu au niveau des bases d'oxydation et des coupleurs permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (c'est-à-dire abîmée) entre sa pointe et sa racine.

La coloration est généralement réalisée en milieu fortement alcalin, en présence de peroxyde d'hydrogène. Toutefois, l'utilisation des milieux alcalins en présence de peroxyde d'hydrogène présente pour inconvénient d'entraîner une dégradation non négligeable des fibres, ainsi qu'une décoloration des fibres kératiniques qui n'est pas toujours souhaitable.

La coloration d'oxydation des fibres kératiniques peut également être réalisée à l'aide de systèmes oxydants différents du peroxyde d'hydrogène tels que des systèmes enzymatiques.

La demande de brevet FR 2769219 décrit l'utilisation d'une enzyme uricase et de son substrat l'acide urique en coloration d'oxydation pour la teinture de fibres kératiniques.

La demande EP-A-0 310 675 décrit l'utilisation de précurseur de colorant d'oxydation de type benzénique en association avec des enzymes telles que la pyranose-oxydase, la glucose-oxydase.

Il est également connu d'utiliser des aldéhydes en coloration capillaire. La demande de brevet WO 01 62219 décrit un agent de teinture des fibres kératiniques obtenu en mélangeant deux composés : d'une part un composé contenant au moins une liaison carbonyle tel qu'un aldéhyde et d'autre part un composé de type indole ou dérivé 3H indolium.

La demande WO 02/47633 est basé sur l'utilisation d'une enzyme oxydante, un alcool aromatique, et des amines primaires ou secondaires, aliphatiques ou aromatiques, des hétérocycles comprenant de l'azote, des acides aminés, des oligopeptides, des aromatiques hydroxylés, des composés ayant des liaisons C-H activées.

Le but de la présente invention est de fournir de nouvelles compositions tinctoriales pour la teinture des fibres kératiniques, et en particulier les fibres kératiniques humaines telles que les cheveux qui soient propres, non toxiques et qui respectent la nature des fibres kératiniques.

De manière avantageuse et inattendue, la demanderesse a découvert qu'il est possible d'atteindre ce but au moyen d'une composition contenant, dans un milieu approprié pour la teinture, au moins un précurseur d'aldéhyde, au moins une enzyme capable de générer un aldéhyde à partir du précurseur d'aldéhyde et au moins une hydrazone hétéroaromatique.

Les compositions tinctoriales selon la présente invention présentent l'avantage de pouvoir être mises en oeuvre en utilisant l'eau comme constituant essentiel du milieu tinctorial. Elles sont ainsi propres, non toxiques et respectent la nature des fibres kératiniques.

Ces compositions sont utilisées selon un procédé de mise en oeuvre simple dans lequel les risques liés à la manipulation de produits fortement réactifs tels que les aldéhydes sont évités. Les aldéhydes fraîchement produits par voie biochimique réagissent ensuite avec d'autres molécules, par conséquent, ces aldéhydes ne s'accumulent pas à l'endroit où ils sont formés.

Les compositions selon la présente invention permettent l'obtention de teintures de couleurs naturelles, peu sélectives et résistantes, ces compositions sont capables d'engendrer de nouveaux colorants pouvant donner des nuances variées de couleur intense et uniforme, sans dégradation significative du cheveu.

La présente demande a encore pour objet un procédé de teinture des fibres kératiniques et en particulier des fibres kératiniques humaines telles que les cheveux, tel qu'on applique sur les fibres kératiniques la composition selon l'invention pendant une durée suffisante pour développer la coloration désirée.

De manière avantageuse, la composition selon l'invention réagit *in situ* sur les fibres kératiniques. Le ou les colorant(s) obtenu(s) pénètrent et teintent la fibre kératinique.

Les précurseurs d'aldéhyde peuvent être choisis parmi les acides aminés tels que la N-6 méthyl lysine, la diméthylglycine, le méthyl glutamate, la thréonine et la sarcosine ; les 2-oxo acides tels que le 2-oxoacidepyruvate, le benzoylformate, le phényl pyruvate ; les alcools primaires en particulier le méthanol, l'éthanol, le benzyl alcool.

Ils sont de préférence choisis parmi les alcools primaires. Les alcools primaires utiles sont les alcools primaires aliphatiques ou aromatiques. A titre d'exemple, on peut citer le méthanol, l'éthanol, le propanol, les alcools primaires à longues chaînes hydrocarbonées par exemple en C₁₂ à C₁₅, l'alcool benzylique, l'alcool 4-amino-benzylique.

Les enzymes capables de générer un aldéhyde à partir du précurseur d'aldéhyde peuvent notamment être choisies parmi les alcool déshydrogenases EC 1.1.1.1, les alcool déshydrogenases EC 1.1.1.2, les alcool déshydrogenases EC 1.1.1.71, les alcool aromatique déshydrogenases EC 1.1.1.90 encore appelées aryl alcool déshydrogenases, les alcool aromatique déshydrogenases EC 1.1.1.97, les alcool 3-hydroxybenzylique déshydrogenases EC 1.1.1.97, les alcool coniferylique déshydrogenases EC 1.1.1.194, les alcool cinnamylique déshydrogenases EC 1.1.1.195, les méthanol déshydrogenases EC 1.1.1.244, les alcool aromatique oxydases EC 1.1.3.7 encore appelées aryl alcool oxydases, les alcool oxydases EC 1.1.3.13, les 4-hydroxymandelate oxydases EC 1.1.3.19, les alcool à longue chaîne hydrocarbonée oxydases EC 1.1.3.20, les méthanol oxydases EC 1.1.3.31, les alcool déshydrogenases EC 1.1.99.20, les sarcosinase oxydases EC 1.5.3.1, les N6-méthyl-lysine oxydases EC 1.5.3.4, les diméthylglycine oxidases EC 1.5.3.10, les sarcosine déshydrogenases EC 1.5.99.1, les diméthylglycine déshydrogenases EC 1.5.99.2, les méthylglutamate déshydrogenases EC 1.5.99.5, les oxoacides décarboxylases EC 4.1.1.1, les benzoylformate décarboxylases EC 4.1.1.7, les phénylpyruvate décarboxylases EC 4.1.1.43, les threonine aldolase EC 4.1.2.5.

L'enzyme capable de générer un aldéhyde à partir du précurseur d'aldéhyde utilisée dans la composition tinctoriale selon l'invention peut être issue d'un extrait de végétaux, d'animaux, de microorganismes (bactérie, champignon, levure, microalgue) ou de virus, de cellules différenciées ou dédifférenciées, obtenues *in vivo* ou *in vitro,* modifiées ou non modifiées génétiquement, ou synthétiques (obtenues par synthèse chimique ou biotechnologique).

A titre d'exemples d'enzymes utiles on peut citer en particulier les genres *Plectranthus, Pinus, Gastropode, Manduca, Pichia, Candida, Pleurotus, Pseudomonas,* et de façon encore plus particulière les espèces suivantes : *Plectranthus colleoides, Pinus strobus* qui est une espèce d'origine végétale, *Gastropode mollusc, Manduca sexta* qui sont d'origine animale, *Pichia pastoris* et *Candida boidinii* qui sont des levures, *Pleurotus pulmonarius* qui est un champignon, et *Pseudomonas pseudoalcaligenes* qui est une bactérie.

Généralement, la concentration de l'enzyme utilisée dans la composition tinctoriale est comprise 0,005% et 40% en poids par rapport au poids total de ladite composition et de préférence comprise entre 0,05% et 10% en poids par rapport au poids de cette composition.

L'activité enzymatique des enzymes utilisées conformément à l'invention peut être définie à partir de l'oxydation du donneur (le précurseur d'aldéhyde) en condition aérobie. Unité U correspond à la quantité d'enzyme conduisant à la génération de 1 µmole d'aldéhyde par minute à un pH donné et à une température de 25°C.

De façon préférentielle, la quantité d'enzyme est comprise entre 0,2 et 4.10⁸ unités U pour 100g de composition tinctoriale.

Le choix de l'enzyme est fonction de la nature du précurseur d'aldéhyde. Par exemple, lorsque le précurseur d'aldéhyde est un alcool, alors l'enzyme est choisie parmi les enzymes capables de générer un aldéhyde à partir de cet alcool. Lorsque le précurseur d'aldéhyde est le méthylglutamate, alors l'enzyme est une méthylglutamate déshydrogénase.

Selon une variante préférée, le précurseur d'aldéhyde est un alcool primaire et l'enzyme est une enzyme capable de générer l'aldéhyde à partir d'un alcool. Par exemple, lorsque l'alcool primaire est un alcool aliphatique en C₁ à C₆, alors l'enzyme capable de générer l'aldéhyde est choisie parmi les alcool oxydases, les alcool déshydrogenases, les méthanol déshydrogenases, les méthanol oxydases. Lorsque l'alcool primaire est l'alcool benzylique, le 4-terbutyl benzylique alcool, le 3-hydroxy-4-méthoxybenzyl alcool, le vératryl alcool, le 4-méthoxybenzyl alcool, l'alcool cinnamique, le 2,4 hexadiéne-1-ol, on peut utiliser comme précurseur d'aldéhyde les aryl alcool oxydases ou les alcool aromatique déshydrogenases.

Généralement, la concentration en précurseur d'aldéhyde est comprise entre 0,01% et 40% en poids par rapport au poids total de la composition et de préférence comprise entre 0,05% et 10% en poids par rapport au poids total de la composition.

Les hydrazones hétéroaromatiques utilisables au sens de la présente demande sont des composés capables de générer une substance colorée par réaction avec un aldéhyde, c'est-à-dire des précurseurs de colorant. Ce sont de préférence des hydrazones hétéroaromatiques qui ont pour formule :

Ar=N-NH₂

dans laquelle Ar est un héterocycle aromatique à 5 ou 6 chaînons comprenant au moins un atome d'azote. Ar est de préférence choisi parmi les hétérocycles suivants : les pyrroles, les pyridines, les pyrazoles, les imidazoles.

Ar peut également être un groupe hétéroaromatique polycyclique condensé à 9 ou 10 chaînons comprenant au moins un atome d'azote de préférence un isoindole.

Ar peut également être substitué sur au moins une position. De préférence, Ar est tel que les atomes d'azote de l'hétérocycle sont substitués par un substituant choisi dans le groupe formé par les alkyles en C₁ à C₄, tels que les groupes méthyle, éthyle, propyles et butyles, les alcools en C₁ à C₄ tels que les groupes méthanol (-CH₂OH), éthanol (-(C₂H₄)OH), propanols (-(C₃H₆)OH), butanols (-(C₂H₈)OH), les éthers en C₁ à C₄, tel que les groupes méthoxy (-OCH₃), éthoxy (-OC₂H₅), propanoxy (-OC₃H₇),butanoxy (-OC₄H₈).

Généralement la concentration en hydrazone hétéroaromatique est comprise entre 0,0005 et 20%, de préférence entre 0,05 et 10% en poids par rapport au poids total de la composition.

La composition selon l'invention peut en outre contenir au moins un autre précurseur de colorant d'oxydation. La nature des précurseurs de colorants (bases et/ou coupleurs) d'oxydation classiques utilisés dans la composition selon l'invention n'est pas critique.

Les bases d'oxydation classiques peuvent notamment être choisies parmi les paraphénylènediamines, les bis-phénylalkylènediamines, les para-aminophénols, les ortho-aminophénols et les bases hétérocycliques et leurs sels d'addition.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, la 4 aminophenyl pyrrolidine, le 2 thiényl paraphénylène diamine, le 2-β hydroxyéthylamino 5-amino toluène et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 3,4-diamino pyridine, et leurs sels d'addition avec un acide.

Parmi les dérivés pyrimidiniques, on peut citer les composés décrits par exemple dans les brevets DE 2 359 399 ; JP 88-169 571 ; JP 05 163 124 ; EP 0 770 375 ou demande de brevet WO 96/15765 comme la 2-hydroxy 4,5,6-triaminopyrimidine, la 2,4-dihydroxy 5,6-diaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Généralement la concentration de cette base d'oxydation est comprise entre 0,0005 et 10% en poids par rapport au poids total de la composition.

Parmi les coupleurs d'oxydation classiques, on peut notamment citer les métaphénylènediamines, les métaaminophénols, les métadiphénols, les coupleurs naphtaléniques et les coupleurs hétérocycliques ainsi que leurs sels d'addition.

A titre d'exemple de coupleurs d'oxydation classiques, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène (ou résorcinol), le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4 méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition.

Généralement la concentration de ce coupleur d'oxydation est comprise entre 0,0001 et 10% en poids par rapport au poids total de la composition.

D'une manière générale, les sels d'addition avec un acide utilisables pour les bases et les coupleurs d'oxydation sont notamment choisis parmi les chlorhydrates, les bromhydrates, les sulfates, les citrates, les succinates, les tartrates, les lactates, les tosylates, les benzènesulfonates, les phosphates et les acétates.

Les sels d'addition utilisables dans le cadre de l'invention sont par exemple choisis parmi les sels d'addition avec la soude, la potasse, l'ammoniaque, les amines ou les alcanolamines.

La composition tinctoriale conforme à l'invention peut en outre contenir un ou plusieurs colorants directs pouvant notamment être choisis parmi les colorants nitrés de la série benzénique, les colorants directs cationiques, les colorants directs azoïques, méthiniques, azométhiniques.

Le milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcools inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensioactifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Ces adjuvants ci-dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 5 et 11 environ, et de préférence entre 7 et 10. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (III) suivante : 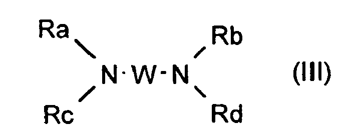 dans laquelle W est un reste propylène éventuellement substitué par un groupement hydroxyle ou un radical alkyle en C₁-C₄ ; Ra, Rb, Rc et Rd, identiques ou différents, représentent un atome d'hydrogène, un radical alkyle en C₁-C₄ ou hydroxyalkyle en C₁-C₄.

La composition tinctoriale peut se présenter sous des formes diverses, telles que sous forme de liquides, de crèmes, de gels, ou sous toute autre forme appropriée pour réaliser une teinture des fibres kératiniques, et notamment des cheveux humains.

Lorsque le(s) précurseur (s) d'aldéhyde et la ou les enzymes sont présents au sein de la même composition prête à l'emploi, ladite composition est de préférence exempte d'oxygène gazeux de manière à éviter toute oxydation prématurée du ou des précurseur (s) d'aldéhyde.

L'invention a également pour objet un procédé de teinture des fibres kératiniques, et en particulier des fibres kératiniques humaines telles que les cheveux, tel qu'on applique sur ces fibres au moins une composition pour la teinture des fibres kératiniques selon l'invention, la durée de cette application étant la durée suffisante pour développer la coloration désirée.

La couleur est alors révélée par l'oxygène de l'air ou à l'aide d'un agent oxydant en fonction de l'enzyme utilisée.

La composition est appliquée sur les fibres kératiniques. Après un temps de pose de 3 à 60 minutes environ, de préférence 5 à 40 minutes environ, les fibres kératiniques sont rincées, lavées au shampooing, rincées à nouveau puis séchées.

Selon la nature de l'enzyme, la composition de l'invention peut être une composition prête à l'emploi qui comprend, dans un milieu approprié pour la teinture des fibres kératiniques, au moins un précurseur d'aldéhyde, au moins une enzyme capable de générer un aldéhyde à partir du précurseur d'aldéhyde et au moins une hydrazone, l'ensemble est alors stocké sous forme anaérobie, exempte d'oxygène gazeux.

Lorsque l'enzyme est capable de générer un aldéhyde à partir du précurseur d'enzyme en l'absence d'un agent oxydant ou de l'oxygène de l'air, alors la composition est stockée sous forme séparée : sous la forme d'une composition (A) comprenant, dans un milieu approprié pour la teinture des fibres kératiniques, au moins un précurseur d'aldéhyde et d'autre part, une composition (B) renfermant, dans un milieu approprié pour la teinture des fibres kératiniques, au moins une enzyme capable de générer un aldéhyde à partir du précurseur d'aldéhyde, la composition (A) et/ou la composition (B) contenant au moins une hydrazone. Le procédé de teinture comprend alors une étape préliminaire qui consiste à procéder au mélange des compositions (A) et (B) au moment de l'emploi et appliquer ce mélange sur les fibres kératiniques.

Le procédé de teinture des fibres kératiniques, et en particulier des fibres kératiniques humaines telles que les cheveux, peut être mis en oeuvre en mélangeant sur les fibres kératiniques la composition selon la présente invention.

La couleur peut être révélée à pH acide, neutre ou alcalin.

Suivant le procédé selon la présente invention, la couleur peut être révélée à l'aide d'un agent oxydant.

L'agent oxydant, lorqu'il est nécessaire, peut être ajouté à la composition de l'invention juste au moment de l'emploi ou il peut être mis en oeuvre à partir d'une composition oxydante le contenant, appliquée simultanément ou séquentiellement à la composition de l'invention.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie entre 5 et 11, de préférence entre 7 et 10. Il peut être ajusté à la valeur désirée au moyen d'agents acidifiants ou alcalinisants habituellement utilisés en teinture des fibres kératiniques et tels que définis précédemment.

La composition qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

Un autre objet de l'invention est un dispositif à plusieurs compartiments ou "kit" de teinture comportant un premier compartiment renferme la composition (A) telle que définie ci-dessus et un deuxième compartiment renferme la composition (B) telle que définie ci-dessus. Ce dispositif peut contenir une troisième composition contenant un agent oxydant, cette troisième composition est de préférence contenue dans un troisième compartiment. Ce dispositif peut être équipé d'un moyen permettant de délivrer sur les cheveux le mélange souhaité, tel que les dispositifs décrits dans le brevet FR-2 586 913 au nom de la demanderesse.

Les exemples qui suivent servent à illustrer l'invention sans toutefois présenter un caractère limitatif.

### EXEMPLES

### Exemple 1

Cet exemple concerne une réaction réalisée *in situ* pour la teinture des fibres kératiniques par conversion enzymatique d'éthanol en acétaldéhyde puis couplage de l'acétaldéhyde avec la N-éthanol pyridine 2-hydrazone.
- La réaction est effectuée à pH compris entre 7 et 9 avec un tampon phosphate à la concentration de 30mM.
- L'alcool oxydase (EC 1.1.3.13) de *Pichia pastoris* est ajouté à la concentration finale de 100U par ml.
- Le précurseur d'aldéhyde utilisé est l'éthanol qui est ajouté de façon à obtenir une concentration finale *in situ* de 10% (v/v).
- La N-éthanol pyridine 2-hydrazone est ajoutée au mélange réactionnel afin d'obtenir une concentration finale *in situ* de 0,3M.
- Ces différents composants sont mélangés, appliqués sur les fibres kératiniques qui sont incubées à 37°C.
- Après 45 minutes de pose, les fibres kératiniques sont lavées puis rincées.

Les fibres kératiniques présentent une coloration bleu-violet.

### Exemple 2

Cet exemple concerne une réaction réalisée *in situ* pour la teinture des fibres kératiniques par conversion enzymatique de méthanol en formaldéhyde puis couplage du formaldéhyde avec la 4-méthoxy-N-méthyl-pyridine-2-hydrazone.
- La réaction est effectuée à pH compris entre 7 et 9 avec un tampon phosphate à la concentration de 30mM.
- L'alcool oxydase (EC 1.1.3.13) de *Candida boidinii* est ajouté à la concentration finale de 100U par ml.
- Le précurseur d'aldéhyde utilisé est le méthanol qui est ajouté de façon à obtenir une concentration finale *in situ* de 10% (v/v).
- La 4-méthoxy-N-méthyl-pyridine-2-hydrazone est ajoutée au mélange réactionnel afin d'obtenir une concentration finale *in situ* de 0,3M.
- Ces différents composants sont mélangés, appliqués sur les fibres kératiniques qui sont incubées à 37°C.
- Après 45 minutes de pose, les fibres kératiniques sont lavées puis rincées.

Les fibres kératiniques présentent une coloration violette.

### Exemple 3

### A-Préparation d'un extrait de Plectranthus colleoides (L)

Le *Plectranthus colleoides* (L) est une plante de la famille des Labiateae.

L'extrait utilisé est un extrait aqueux brut de *Plectranthus colleoides* (L) réalisé à partir de la plante entière.

La partie aérienne de la plante (les feuilles et les tiges) a été prélevée, séchée à 45°C, broyée sur broyeur à couteaux, puis la poudre obtenue a été tamisée sur un tamis de mailles égales à 0,5µm.

La poudre est extraite par agitation en présence d'eau carbonatée à pH 9,5 préparée comme suit : du carbonate anhydre de sodium est ajouté à 1g/l à de l'eau distillée, le pH est ajusté à 9,5 par addition d'HCl IN.

L'extraction est réalisée à température ambiante à raison de 5g de poudre de plante pour 100ml d'eau carbonatée, placé en agitation pendant 1h30 à 900 tours/mn.

Le mélange est ensuite filtré sous vide sur filtre de 2,7µm de porosité.

Le filtrat obtenu est ensuite congelé puis lyophilisé.

### B-Préparation de la composition tinctoriale

L'extrait de plante est mis en contact avec 0,2% d'une hydrazone décrite ci-dessous mise en solution dans 100 ml de mélange alcool/eau (2/5). La quantité d'extrait est comprise entre 0,3 et 0,4%. A ce mélange sont ajoutés 0,5ml d'eau oxygénée à 3% et 0,5ml de tampon ammoniacal pH 9. La composition est mise en contact avec le cheveu et laissée à température ambiante. Une coloration violette se développe et teinte la fibre kératinique.

La même réaction se développe également sans la présence d'eau oxygénée à pH 7.

Les résultats de coloration sont regroupés dans le tableau suivant :

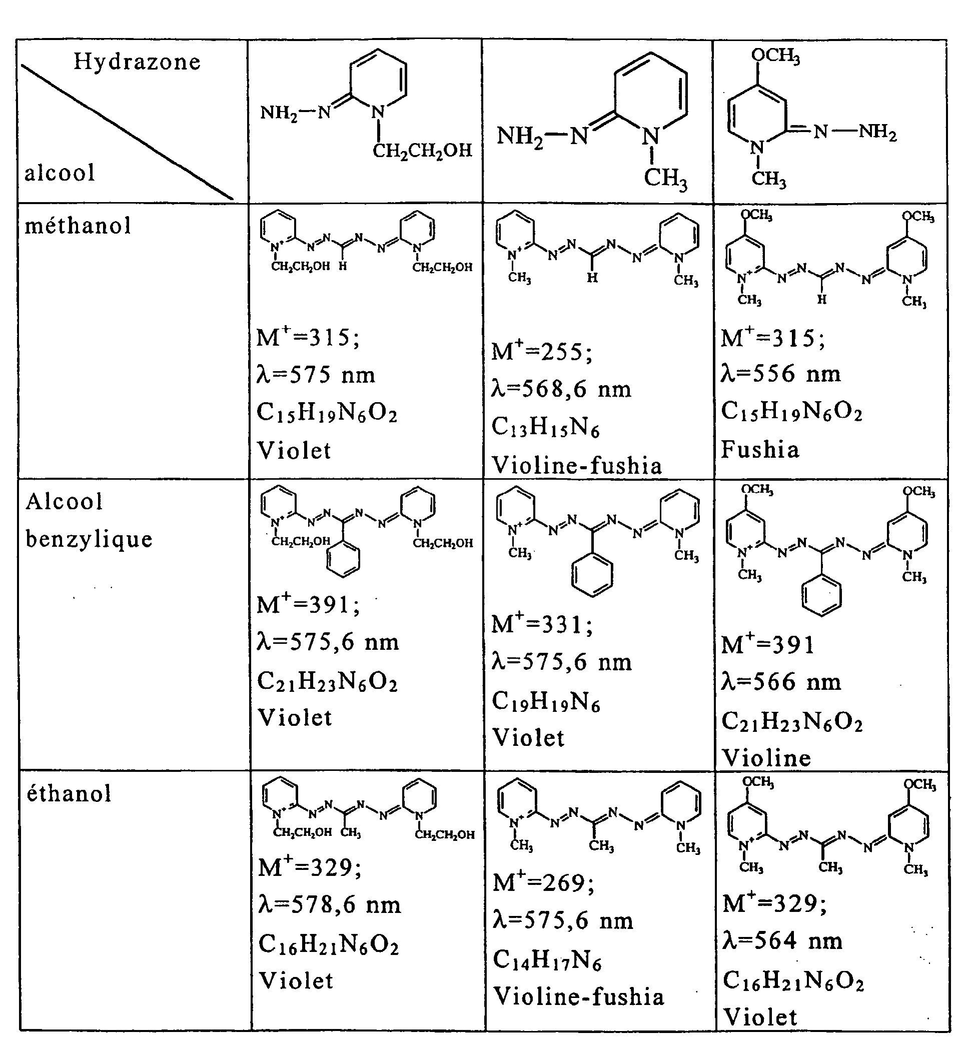

L'analyse des colorants a été réalisée par HPLC, spectroscopie de masse , et RMN.

## Revendications

1. Composition tinctoriale pour la teinture des fibres kératiniques contenant, dans un milieu approprié pour la teinture, au moins un précurseur d'aldéhyde, au moins une enzyme capable de générer un aldéhyde à partir du précurseur d'aldéhyde et au moins une hydrazone hétéroaromatique capable de générer une substance colorée par réaction avec un aldéhyde.

2. Composition selon la revendication 1 telle que l'enzyme est issue d'un extrait choisi parmi les extraits de végétaux, d'animaux, de microorganismes ou de virus, de cellules différenciées ou dédifférenciées, obtenues *in vivo* ou *in vitro,* modifiées ou non modifiées génétiquement, ou synthétiques.

3. Composition selon la revendication 2 telle que l'enzyme est choisie parmi les genres *Plectranthus*, *Pinus, Gastropode, Manduca, Pichia, Candida, Pleurotus, Pseudomonas*, et de façon encore plus particulière les espèces suivantes *Plectranthus colleoides, Pinus strobus, Gastropode mollusc, Manduca sexta, Pichia pastoris, Candida boidinii, Pleurotus pulmonarius, et Pseudomonas pseudoalcaligenes.*

4. Composition selon l'une des revendications 1 à 3, telle que la concentration en enzyme est comprise entre 0,005% et 40% en poids par rapport au poids total de ladite composition.

5. Composition selon l'une des revendications 1 à 4 telle que le précurseur d'aldéhyde est choisi parmi les acides aminés tels que la N-6 méthyl lysine, la diméthylglycine, le méthyl glutamate, la thréonine et la sarcosine ; les 2-oxo acides tels que le 2-oxoacidepyruvate, le benzoylformate, le phényl pyruvate ; les alcools primaires en particulier le méthanol, l'éthanol, le benzyl alcool.

6. Composition selon la revendication 5 telle que la concentration en précurseur d'aldéhyde est comprise entre 0,01% et 40% en poids par rapport au poids total de la composition.

7. Composition selon l'une des revendications de 1 à 6 telle que l'hydrazone hétéroaromatique est une hydrazone de formule:
Ar=N-NH₂
dans laquelle Ar est un hétérocycle à 5 ou 6 chaînons comprenant au moins un atome d'azote ou un groupe hétéroaromatique polycyclique condensé à 9 ou 10 chaînons comprenant au moins un atome d'azote, Ar étant non substitué ou substitué sur les atomes d'azote par un substituant choisi dans le groupe formé par les alkyles en C₁ à C₄, les alcools en C₁ à C₄, les éthers en C₁ à C₄.

8. Composition selon la revendication 7 telle que la concentration en hydrazone hétéroaromatique est comprise entre 0,0005 et 20% en poids par rapport au poids total de la composition.

9. Composition selon l'une des revendications 1 à 8 telle qu'elle comprend une base d'oxydation classique choisie parmi les paraphénylènediamines, les bis-phénylalkylènediamines, les para-aminophénols, les ortho-aminophénols, les bases hétérocycliques et leurs sels d'addition.

10. Composition selon la revendication 9 telle que la concentration de cette base d'oxydation classique est comprise entre 0,0005 et 10% en poids par rapport au poids total de la composition.

11. Composition selon l'une des revendications 1 à 10 telle que la composition comprend un coupleur d'oxydation classique choisi parmi les métaphénylènediamines, les métaaminophénols, les métadiphénols, les coupleurs naphtaléniques, les coupleurs hétérocycliques ainsi que leurs sels d'addition.

12. Composition selon la revendication 11 telle que la concentration de ce coupleur est comprise entre 0,0001 et 10 % en poids par rapport au poids total de la composition.

13. Composition selon l'une des revendications précédentes, **caractérisée par le fait qu'**elle renferme un ou plusieurs colorants directs.

14. Composition selon l'une des revendications précédentes, **caractérisée par le fait qu'**elle renferme un agent oxydant.

15. Procédé de teinture des fibres kératiniques et en particulier des fibres kératiniques humaines telles que les cheveux, **caractérisée par le fait qu'**on applique sur lesdites fibres au moins une composition selon l'une des revendications 1 à 14, pendant une durée suffisante pour développer la coloration désirée.

16. Procédé selon la revendication 15, **caractérisé par le fait que** la composition est une composition prête à l'emploi stockée sous forme anaérobie, exempte d'oxygène gazeux.

17. Procédé selon la revendication 16, **caractérisée par le fait qu'**il comporte une étape préliminaire consistant à mélanger une composition (A) comprenant, dans un milieu approprié pour la teinture, au moins un précurseur d'aldéhyde et une composition (B) renfermant, dans un milieu approprié pour les fibres kératiniques, au moins une enzyme capable de générer un aldéhyde à partir du précurseur d'aldéhyde, la composition (A) et/ou la composition (B) contenant au moins une hydrazone capable de générer une substance colorée par réation avec un aldéhyde puis à appliquer ce mélange sur les puis à appliquer ce mélange sur les fibres kératiniques.

18. Procédé selon l'une des revendications 15 à 17 **caractérisé en ce que** la couleur est révélée à l'aide d'un agent oxydant.

19. Procédé de teinture des fibres kératiniques **caractérisée par le fait qu'**on mélange sur les fibres kératiniques la composition selon l'une des revendications 1 à 14.

20. Dispositif à plusieurs compartiments ou « Kit » de teinture, **caractérisé par le fait qu'**il comporte un premier compartiment renfermant la composition (A) telle que définie dans la revendication 17 et un second compartiment renfermant la composition (B) telle que définie dans la revendication 17.

## Claims

1. Dye composition for dyeing keratin fibres containing, in an appropriate dyeing medium, at least one aldehyde precursor, at least one enzyme able to generate an aldehyde from the aldehyde precursor and at least one heteroaromatic hydrazone capable of generating a coloured substance by reaction with an aldehyde.

2. Composition according to claim 1 such that the enzyme is derived from an extract chosen from among plant, animal, microorganism or virus extracts, from differentiated or dedifferentiated cells, obtained *in vivo* or *in vitro*, whether or not genetically modified, or synthetic.

3. Composition according to claim 2 such that the enzyme is chosen from among the species *Plectranthus Pinus, Gastropoda, Manduca, Pichia, Candida, Pleurotus, Pseudomonas,* and more particularly from among the following species: *Plectranthus colleoides, Pinus strobus, Gastropoda mollusca, Manduca sexta, Pichia pastoris, Candida boidinii, Pleurotus pulmonarius and Pseudomonas pseudoalcaligenes.*

4. Composition according to any of claims 1 to 3, such that the enzyme concentration lies between 0.005 % and 40 % by weight relative to the total weight of said composition.

5. Composition according to any of claims 1 to 4 such that the aldehyde precursor is chosen from among the amino acids such as N-6 methyl lysine, dimethylglycine, methyl glutamate, threonine and sarcosine; the 2-oxo acids such as 2-oxoacidpyruvate, benzoylformate, phenyl pyruvate; the primary alcohols in particular methanol, ethanol, benzyl alcohol.

6. Composition according to claim 5 such that the concentration of aldehyde precursor lies between 0.01 % and 40 % by weight relative to the total weight of the composition.

7. Composition according to any of claims 1 to 6 such that the heteroaromatic hydrazone is a hydrazone having the formula:
AR=N-NH₂
where Ar is a heterocycle with 5 or 6 chain links comprising at least one nitrogen atom or a condensed polycyclic heteroaromatic group with 9 or 10 chain links comprising at least one nitrogen atom, Ar being non-substituted or substituted on the nitrogen atoms by a substituent chosen from the group made up of C₁ to C₄ alkyls, C₁ to C₄ alcohols, C₁ to C₄ ethers.

8. Composition according to claim 7 such that the concentration of heteroaromatic hydrazone lies between 0.0005 and 20 % by weight relative to the total weight of the composition.

9. Composition according to any of claims 1 to 8 such that it contains a conventional oxidation base chosen from among the paraphenylenediamines, bis-phenylalkylenediamines, para-aminophenols, ortho-aminophenols, heterocyclic bases and their addition salts.

10. Composition according to claim 9 such that the concentration of this conventional oxidation base lies between 0.0005 and 10 % by weight relative to the total weight of the composition.

11. Composition according to any of claims 1 to 10 such that the composition comprises a conventional oxidation coupler chosen from among the metaphenylenediamines, metaaminophenols, metadiphenols, naphthalene couplers, heterocyclic couplers and their addition salts.

12. Composition according to claim 11 such that the concentration of this coupler lies between 0.0001 and 10 % by weight relative to the total weight of the composition.

13. Composition according to any of the preceding claims **characterized in that** it contains one or more direct dyes.

14. Composition according to any of the preceding claims **characterized in that** it contains an oxidizing agent.

15. Method for dyeing keratin fibres, human keratin fibres in particular such as hair, **characterized in that** at least one composition according to any of claims 1 to 14 is applied to said fibres for a sufficient length of time to develop the desired colouring.

16. Method according to claim 15 **characterized in that** the composition is a ready-to-use composition stored in anaerobic form free of gaseous oxygen.

17. Method according to claim 16 **characterized in that** it comprises a preliminary step consisting of mixing composition (A) containing, in an appropriate dyeing medium, at least one aldehyde precursor, and composition (B) containing, in an appropriate medium for keratin fibres, at least one enzyme able to generate an aldehyde from the aldehyde precursor, composition (A) and/or composition (B) containing at least one hydrazone capable of generating a coloured substance by reaction with an aldehyde, then applying this mixture to the keratin fibres.

18. Method according to any of claims 15 to 17 **characterized in that** the colour is developed using an oxidizing agent.

19. Method for dyeing keratin fibres **characterized in that** the composition according to any of claims 1 to 14 is mixed on the keratin fibres.

20. Device with several compartments or dye "Kit" **characterized in that** it comprises a first compartment containing composition (A) such as defined in claim 17 and a second compartment containing composition (B) such as defined in claim 17.

## Patentansprüche

1. Farbmittelzusammensetzung zum Färben von Keratinfasern, die in einem zum Färben geeigneten Medium mindestens einen Aldehydvorläufer, mindestens ein Enzym, das befähigt ist, ausgehend von dem Aldehydprecursor einen Aldehyden zu bilden, und mindestens ein heteroaromatisches Hydrazon enthält, das befähigt ist, durch Reaktion mit einem Aldehyden eine farbige Substanz zu bilden.

2. Zusammensetzung nach Anspruch 1, wobei das Enzym aus einem Extrakt stammt, der unter den Pflanzenextrakten, tierischen Extrakten, Extrakten von Mikroorganismen oder Viren, Extrakten von differenzierten oder undifferenzierten Zellen ausgewählt ist, welche *in vivo* oder *in vitro* erhalten werden, genetisch modifiziert oder nicht modifiziert sind, oder synthetisch sind.

3. Zusammensetzung nach Anspruch 2, wobei das Enzym unter den Gattungen *Plectranthus, Pinus, Gastropode, Manduca, Pichia, Candida, Pleurotus* und *Pseudomonas* und insbesondere den folgenden Arten ausgewählt ist: *Plectranthus colleoides, Pinus strobus, Gastropode mollusc, Manduca sexta, Pichia pastoris, Candida boidinii, Pleurotus pulmonarius* und *Pseudomonas pseudoalcalige*nes.

4. Zusammensetzung nach einem der Ansprüche 1 bis 3, wobei die Konzentration des Enzyms im Bereich von 0,005 bis 40 Gew.-%, bezogen auf das Gesamtgewicht der Zusammensetzung, liegt.

5. Zusammensetzung nach einem der Ansprüche 1 bis 4, wobei der Aldehydvorläufer unter den Aminosäuren, wie N-6-Methyllysin, Dimethylglycin, Methylglutamat, Threonin und Sarcosin; 2-Oxosäuren, wie 2-Oxopyruvat, Benzoylformat, Phenylpyruvat; und primären Alkoholen ausgewählt ist, insbesondere Methanol, Ethanol, Benzylalkohol.

6. Zusammensetzung nach Anspruch 5, wobei die Konzentration des Aldehydvorläufers im Bereich von 0,01 bis 40 Gew.-%, bezogen auf das Gesamtgewicht der Zusammensetzung, liegt.

7. Zusammensetzung nach einem der Ansprüche 1 bis 6, wobei das heteroaromatische Hydrazon ein Hydrazon der Formel
Ar=N-NH₂
ist, worin Ar einen 5- oder 6-gliedrigen Heterocyclus bedeutet, der mindestens ein Stickstoffatom enthält, oder eine 9- oder 10-gliedrige, kondensierte, polycyclische heteroaromatische Gruppe, die mindestens ein Stickstoffatom enthält, wobei Ar unsubstituiert vorliegt oder an den Stickstoffatomen mit einem Substituenten substituiert ist, der unter den C₁₋₄-Alkylgruppen, C₁₋₄-Alkoholen und C₁₋₄-Ethern ausgewählt ist.

8. Zusammensetzung nach Anspruch 7, wobei die Konzentration des heteroaromatischen Hydrazons im Bereich von 0,0005 bis 20 Gew.-%, bezogen auf das Gesamtgewicht der Zusammensetzung, liegt.

9. Zusammensetzung nach einem der Ansprüche 1 bis 8, die eine herkömmliche Oxidationsbase enthält, die unter den *p*-Phenylendiaminen, Bisphenylalkylendiaminen, *p*-Aminophenolen, o-Aminophenolen, heterocyclischen Basen und deren Additionssalzen ausgewählt ist.

10. Zusammensetzung nach Anspruch 9, wobei die Konzentration der herkömmlichen Oxidationsbase im Bereich von 0,0005 bis 10 Gew.-%, bezogen auf das Gesamtgewicht der Zusammensetzung, liegt.

11. Zusammensetzung nach einem der Ansprüche 1 bis 10, wobei die Zusammensetzung einen herkömmlichen Kuppler enthält, der unter den *m*-Phenylendiaminen, *m*-Aminophenolen, *m-*Dihydroxybenzolen, Naphthalinkupplern, heterocyclischen Kupplern und deren Additionssalzen ausgewählt ist.

12. Zusammensetzung nach Anspruch 11, wobei die Konzentration des Kupplers im Bereich von 0,0001 bis 10 Gew.-%, bezogen auf das Gesamtgewicht der Zusammensetzung, liegt.

13. Zusammensetzung nach einem der vorhergehenden Ansprüche, **dadurch gekennzeichnet, dass** sie einen oder mehrere Direktfarbstoffe enthält.

14. Zusammensetzung nach einem der vorhergehenden Ansprüche, **dadurch gekennzeichnet, dass** sie ein Oxidationsmittel enthält.

15. Verfahren zum Färben von Keratinfasern und insbesondere menschlichen Keratinfasern, wie Haaren, **dadurch gekennzeichnet, dass** auf die Fasern mindestens eine Zusammensetzung nach einem der Ansprüche 1 bis 14 während einer Zeitspanne aufgebracht wird, die ausreicht, um die gewünschte Färbung zu bilden.

16. Verfahren nach Anspruch 15, wobei die Zusammensetzung eine gebrauchsfertige Zusammensetzung ist, die in anaerober Form ohne gasförmigen Sauerstoff aufbewahrt wird.

17. Verfahren nach Anspruch 16, **dadurch gekennzeichnet, dass** es einen vorbereiteten Schritt umfasst, der darin besteht, eine Zusammensetzung (A), die in einem zum Färben geeigneten Medium mindestens einen Aldehydvorläufer enthält, und eine Zusammensetzung (B) zu vermischen, die in einem für Keratinfasern geeigneten Medium mindestens ein Enzym enthält, das aus dem Aldehydvorläufer einen Aldehyden bilden kann, wobei die Zusammensetzung (A) und/oder die Zusammensetzung (B) mindestens ein Hydrazon enthalten, das befähigt ist, durch Reaktion mit einem Aldehyden eine farbige Substanz zu bilden, worauf dieses Gemisch auf die Keratinfasern aufgebracht wird.

18. Verfahren nach einem der Ansprüche 15 bis 17, **dadurch gekennzeichnet, dass** die Farbe mit Hilfe eines Oxidationsmittels gebildet wird.

19. Verfahren zum Färben von Keratinfasern, **dadurch gekennzeichnet, dass** die Zusammensetzung nach einem der Ansprüche 1 bis 14 auf den Keratinfasern vermischt wird.

20. Vorrichtung mit mehreren Abteilungen oder «Kit» zum Färben, **dadurch gekennzeichnet, dass** sie eine erste Abteilung mit einer Zusammensetzung (A), wie sie in Anspruch 17 definiert wurde, und eine zweite Abteilung mit der Zusammensetzung (B), wie sie in Anspruch 17 definiert wurde, enthält.
